# EUROPEAN PATENT APPLICATION

(11) **EP 2 716 759 A1**
(43) Date of publication of application: **09.04.2014**
(21) Application number: 12792838.0
(22) Date of filing: 24.05.2012
(51) Int. Cl.: C12N 15/09, C12N 1/15, C12N 1/19, C12N 1/21, C12N 5/10

(54) **GENE TARGETING VECTOR, METHOD FOR MANUFACTURING SAME, AND METHOD FOR USING SAME**

(30) Priority: 27.05.2011 JP 2011118564
(71) Applicant: Public University Corporation Yokohama City University, Yokohama-shi, Kanagawa 236-0027 (JP)
(72) Inventor: Adachi, Noritaka, Yokohama-shi Kanagawa 236-0027 (JP)
(74) Representative: Ford, Hazel
(86) International application number: PCT/JP2012/063248
(87) International publication number: WO 2012/165270

(57) **Abstract**

Provided is a gene targeting vector capable of highly efficient gene targeting.

A gene targeting vector in which a DNA sequence allowing for bicistronic expression is present 5' upstream of a selection marker. A method for producing a gene targeting vector, comprising linking a DNA fragment homologous to a 5' upstream region of a target site, a selection marker having a DNA sequence allowing for bicistronic expression present 5' upstream thereof, and a DNA fragment homologous to a 3' downstream region of the target site.

SEQUENCE LISTING

<110>  Pubilic University Corporation Yokohama City University

<120>  Gene-targeting vectors, a method of preparing the same and a method
of using the same

<130>  FP-168PCT

<150>  JP P2011-118564
<151>  2011-05-27

<160>  6

<170>  PatentIn version 3.1

<210>  1
<211>  50
<212>  DNA
<213>  Artificial

<220>
<223>  HPRT 5' Fw primer

<400>  1
ggggacaact ttgtatagaa aagttgcaca tcacaggtac catatcagtg          50

<210>  2
<211>  50
<212>  DNA
<213>  Artificial

<220>
<223>  HPRT 5' Rv primer

<400>  2
ggggactgct tttttgtaca aacttgcaca tctcgagcaa gacgttcagt          50

<210>  3
<211>  58
<212>  DNA
<213>  Artificial

<220>
<223>  HPRT 3' Fw primer

<400>  3
ggggacagct ttcttgtaca aagtggcctg caggatcaca ttgtagccct ctgtgtgc          58

<210>  4
<211>  80
<212>  DNA
<213>  Artificial

<220>
<223>  HPRT 3' Rv primer

<400>  4
ggggacaact ttgtataata aagttgctat attaccctgt tatccctagc gtaactcagg          60

gtagaaatgc tacttcaggc                                                    80

<210>  5
<211>  24
<212>  DNA
<213>  Artificial

<220>
<223>  HPRT-F primer

<400>  5
tgagggcaaa ggatgtgtta cgtg                                               24

<210>  6
<211>  24
<212>  DNA
<213>  Artificial

<220>
<223>  HPRT-R primer

<400>  6
ttgatgtaat ccagcaggtc agca                                               24

## Description

### Technical Field

The present invention relates to a gene targeting vector, a method for producing the same, and a method for using the same.

### Background Art

It is possible to disrupt a gene(s) on the genome or replace it with a transfected DNA fragment by utilizing cell's ability for homologous recombination (Non-Patent Documents 1 and 2). This technique is referred to as gene targeting. This technique has not only been a powerful tool for the analyses of the functions of individual genes, but it is also anticipated to be used as an ideal gene therapy or breeding method (Non-Patent Document 3). However, the efficiency of such gene targeting in common higher animal or plant cells is very low, and thus, it has been desired to develop an improved method that copes with this difficulty. With the use of a promoterless-type targeting vector (including an exon-trapping-type targeting vector), an increase in the targeting efficiency can be expected (Non-Patent Documents 4 and 5). However, since a gene with a low expression level in the cell (a gene with a low promoter activity) is less likely to be trapped with a common IRES sequence, it has been desired to develop an improved method that solves this problem.

### Citation List

### Non Patent Literature

Non Patent Literature 1: Capecchi, MR (1989) Altering the genome by homologous recombination. Science 244: 1288-1292
Non Patent Literature 2: Vasquez KM, Marburger K, Intody Z, et al. (2001) Manipulating the mammalian genome by homologous recombination. Proc. Natl. Acad. Sci. USA 98: 8403-8410
Non Patent Literature 3: Yanez RJ, Porter AC (1998) Therapeutic gene targeting. Gene Ther 5: 149-159
Non Patent Literature 4: Bunz F, Dutriaux A, Lengauer C, et al. (1998) Requirement for p53 and p21 to Sustain G2 Arrest After DNA Damage. Science 282: 1497-1501
Non Patent Literature 5: Adachi N, So S, Iiizumi S, et al. (2006) The human pre-B cell line Nalm-6 is highly proficient in gene targeting by homologous recombination. DNA Cell Biol. 25: 19-24

### Disclosure of the Invention

### Problems to be Solved by the Invention

An object of the present invention is to provide a gene targeting vector capable of highly efficient gene targeting.

In addition, another object of the present invention is to provide a method for producing a gene targeting vector capable of highly efficient gene targeting.

Moreover, it is a further object of the present invention to provide a method for producing a gene knockout cell with the use of a gene targeting vector capable of highly efficient gene targeting.

### Means for Solving the Problems

The biggest cause of the problem of very low efficiency of gene targeting is that a targeting vector introduced into a cell is inserted into a random site on the genome at a high frequency (random integration). However, with the use of a promoterless-type targeting vector, an increase in targeting efficiency can be expected. Thus, if such a vector, in particular, an exon-trapping-type targeting vector can be produced simply and promptly, certain technological innovation should be achieved. In fact, however, when an IRES sequence is used, the expression level of a marker gene used in the selection becomes lower than the expression level of the gene on the genome and this would be the reason for the difficulty in trapping a gene with a low expression level.

The present inventor developed a method for producing an exon-trapping-type targeting vector simply and promptly by utilizing the MultiSite Gateway System of Invitrogen (that needs neither a restriction enzyme treatment nor a DNA ligation reaction). In this method, the designing of PCR primers to be used for amplification of homologous region arms would be an important key. Ultra-highly efficient gene targeting becomes possible by applying the thus produced vector to human lymphocytes. In addition, by performing gene targeting according to exon trapping using a 2A peptide sequence, the expression of a gene on the genome can be maintained at the same level as the expression of the marker gene used. Thus, it is anticipated that the trapping of a gene with a low expression level will become easier to achieve than before. Moreover, the present inventor also developed a method for constructing such a vector simply and promptly.

A summary of the present invention is as follows.
(1) A method for producing a gene targeting vector, comprising linking a DNA fragment homologous to a 5' upstream region of a target site, a selection marker having a DNA sequence allowing for bicistronic expression present 5' upstream thereof, and a DNA fragment homologous to a 3' downstream region of the target site.
(2) The method according to (1) above, wherein the DNA fragment homologous to the 5' upstream region of the target site comprises a splice acceptor site.
(3) The method according to (1) or (2) above, wherein the DNA fragment homologous to the 3' downstream region of the target site or the DNA fragment homologous to the 5' upstream region of the target site comprises a restriction site(s) for linearization.
(4) A gene targeting vector, wherein a DNA sequence allowing for bicistronic expression is present 5' upstream of a selection marker.
(5) The vector according to (4) above, wherein the selection marker has a poly A sequence but does not have a promoter.
(6) The vector according to (4) or (5) above, wherein the selection marker is flanked with target sequences of a site-specific recombinant enzyme.
(7) The vector according to any one of (4) to (6) above, further comprising a splice acceptor site.
(8) The vector according to any one of (4) to (7) above, further comprising a restriction site(s) for linearization.
(9) A method for producing a gene knockout cell, comprising introducing a genetic mutation into a cell with the use of the gene targeting vector according to any one of (4) to (8) above.
(10) A vector comprising a selection marker to be used for the production of a gene targeting vector, wherein a DNA sequence allowing bicistronic expression is incorporated 5' upstream of the selection marker.
(11) The vector according to (10) above, further comprising a splice acceptor site.
(12) A vector comprising a selection marker to be used for the production of a gene targeting vector, further comprising sites for incorporation of a DNA fragment homologous to a 5' upstream region of a target site and a DNA fragment homologous to a 3' downstream region of the target site, and a restriction site(s) for linearization.

### Advantages of the Invention

It has become possible to produce an exon-trapping-type targeting vector much more simply and promptly than before. In addition, it has become possible to perform ultra-highly efficient gene targeting in human cells. Moreover, if it becomes possible to easily trap a gene with a low expression level, it would be easy to apply such gene targeting to genes that are expressed at low levels. Accordingly, such gene targeting is effective for enabling a wider and/or more efficient use of gene knockout or gene trapping in the fields of basic biology, medicine, and agriculture & livestock industries.

The present specification includes part or all of the contents as disclosed in the specification and/or drawings of Japanese Patent Application No. 2011-118564 based on which the present application claims priority.

### Brief Description of the Drawings

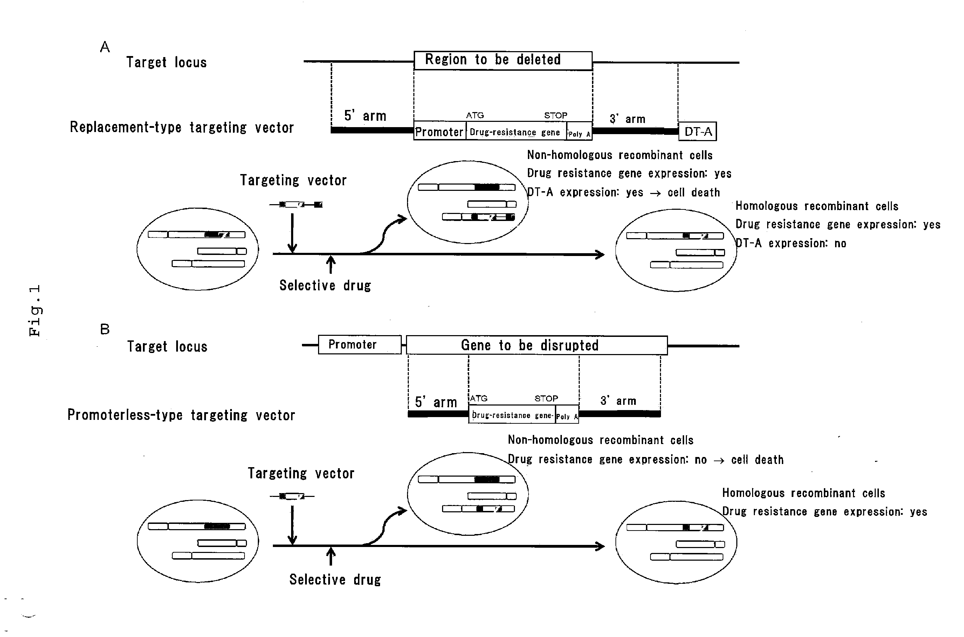

Figure 1 shows a structure of a targeting vector. Figure 1A shows a structure of a common substitution-type targeting vector. When a targeting vector is introduced into cells and colonies are allowed to form in the presence of a selective drug, homologous recombinant cells can be obtained in which the target site is replaced with a drug resistance gene, and non-homologous recombinant cells in which the targeting vector is inserted into a random site(s) on the chromosome. The non-homologous recombinant cells make up an overwhelming majority. That is to say, since both the homologous recombinant cells and the non-homologous recombinant cells have the drug resistance gene, it is difficult to obtain homologous recombinant cells by this drug selection only. However, if a suicide gene such as DT-A is added to the outside of either arm, non-homologous recombinant cells will die due to the expression of the suicide gene incorporated into the chromosome. Each ellipse in the figure indicates a cell, and the rectangular box like a bar in the ellipse indicates a chromosome. The thick gray region in the chromosome indicates a target site, and the light gray regions in the chromosome and the targeting vector indicate homologous regions. The region flanked with the arms of the targeting vector indicates a drug resistance gene, and the black square region indicates DT-A. Figure 1B shows an example of the structure of a promoterless-type targeting vector. Differing from the aforementioned substitution-type targeting vector, a gene to be used as a positive selection marker does not have its own promoter. Thus, theoretically, only when gene targeting by homologous recombination takes place, a target gene promoter on the chromosome is used, and the expression of a marker gene begins;

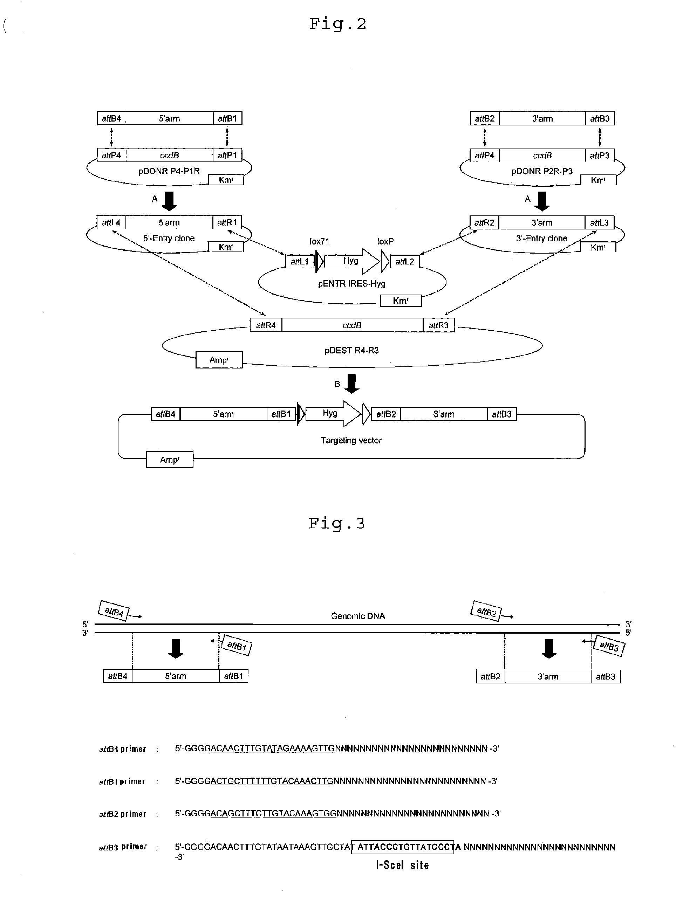

Figure 2 shows an outline of the production of an exon-trapping-type targeting vector by employing Multisite Gateway technology. A 5' arm and a 3' arm each having attB sequences at both ends are amplified by PCR, and a 5'-entry clone and a 3'-entry clone are then produced by BP recombination reaction (Figure 2A). Using the two entry clones thus obtained, as well as pENTR IRES-Hyg and pDEST R4-R3, a targeting vector is produced by LR recombination (Figure 2B). The symbol "Hyg" indicates a hygromycin resistance gene, "DT-A" indicates a diphtheria toxin A fragment gene, "Km^{r}" indicates a kanamycin resistance gene, and "Amp^{r}" indicates an ampicillin resistance gene;
Figure 3 shows an outline of PCR to be performed for amplification of arms and it also shows primer sequences. Each arm is amplified by PCR such that it is flanked with attB sequences. The underlined portions in the primer sequences indicate respective att sequences, N indicates a template-specific sequence, and the framed portion indicates an I-SceI recognition sequence. The template-specific sequence may have a length of approximately 25 nucleotides;

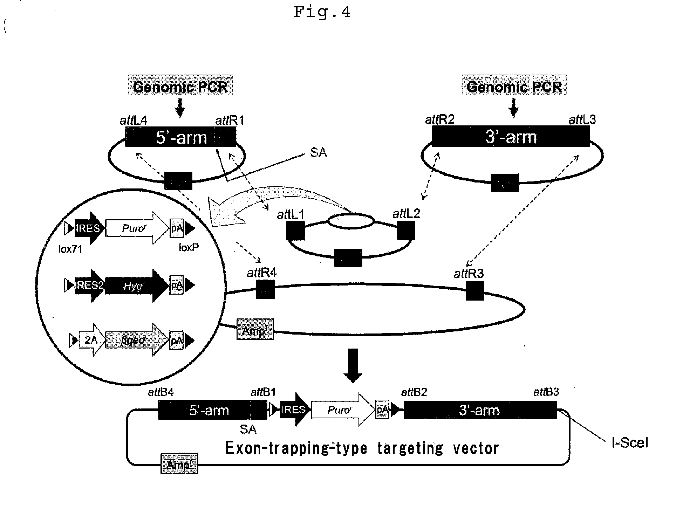

Figure 4 shows an outline of the production of an exon-trapping-type targeting vector by employing Multisite Gateway technology. The SA site (splice acceptor site) is contained in the 5' arm. In addition, the selection marker has a poly A sequence (pA). The symbol "Km^{r}" indicates a kanamycin resistance gene, "Hyg^{r}" indicates a hygromycin resistance gene, "βgeo^{r}" indicates a fusion gene of a β-galactosidase gene with a neomycin resistance gene, "IRES" indicates an IRES sequence, "2A" indicates a 2A peptide sequence, "IRES2" indicates an IRES2 sequence, "Amp^{r}" indicates an ampicillin resistance gene, and "Puro^{r}" indicates a puromycin resistance gene; and

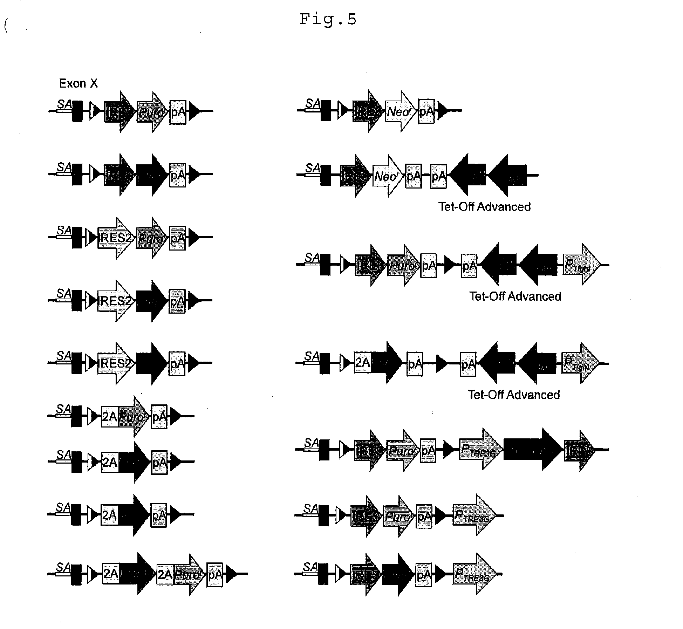

Figure 5 shows various types of selection markers as constructed. Selection markers which correspond to those in the circle in the vector of Figure 4 are provided in an available state. The symbol "Exon X" indicates a target exon, "SA" indicates an SA site, "IRES" indicates an IRES sequence, "Puro^{r}" indicates a puromycin resistance gene, "pA" indicates a poly A sequence, "Hyg^{r}" indicates a hygromycin resistance gene, "IRES2" indicates an IRES2 sequence, "β-geo" indicates a fusion gene of a β-galactosidase gene with a neomycin resistance gene, "2A" indicates a 2A peptide sequence, "EGFP" indicates an enhanced green fluorescent protein gene, "Neo^{r}" indicates a neomycin resistance gene, "tTA2^{s}" indicates a tetracycline-controllable transcription factor gene, "P_{CMV}" indicates a CMV promoter, "Tet-Off Advanced" indicates a gene expression control system using tetracycline (the system is commercially available from TAKARA BIO INC.), "P_{Tight}" indicates a tetracycline-controllable promoter, "P_{TRE3G}" indicates a TRE3G promoter, and "mCherry" indicates a red fluorescent protein gene.

### Best Mode for Carrying Out the Invention

Hereinafter, the embodiments of the present invention will be described more in detail.

Gene targeting is a technique of introducing a mutation into any given site on the chromosome by utilizing a homologous recombination mechanism. However, the homologous recombination frequency is low in higher organisms. In general, the frequency at which a targeting vector is randomly inserted into an incorrect site in a cell is 100 or more times higher than the frequency at which it is inserted in a target site in the cell. Hence, in order to efficiently select and obtain homologous recombinant cells, some modifications need to be made on the targeting vector. The most commonly used substitution-type targeting vector has such a structure that a positive selection marker (corresponding to the "selection marker" in the present invention) is flanked with DNA fragments that are homologous to a 5' upstream region and a 3' downstream region of a target site (a region to be deleted) (the fragments are hereinafter sometimes referred to as a "5' arm" and a "3' arm," respectively) (Figure 1A). Examples of the positive selection marker include: drug resistance genes such as a hygromycin resistance gene, a puromycin resistance gene, a neomycin resistance gene, and β-geo (a fusion gene of a β-galactosidase gene with a neomycin resistance gene); fluorescent protein genes such as a green fluorescent protein (GFP) gene, an enhanced green fluorescent protein (enhanced GFP; EGFP) gene, and a red fluorescent protein (mCherry) gene; and a luciferase gene. Since the target site is replaced with a positive selection marker upon homologous recombination, recombinant cells can be selected using this marker as an indicator. However, the expression of a marker gene does occur even if the marker is inserted randomly by non-homologous recombination. Hence, a common practice for removing non-homologous recombinant cells is to add a gene for negative selection to the outside of the arm in the targeting vector. Examples of such a gene for negative selection include suicide genes such as HSV-TK and DT-A. As an alternative method, a promoterless method (including an "exon trapping method") has been developed, in which a drug resistance gene (selection marker) does not have a promoter (Figure 1B). In this method, when homologous recombination takes place, the expression of a positive selection marker gene begins.

In the present specification, a method for producing a substitution-type targeting vector with the use of Multisite Gateway technology (Iiizumi, S, Nomura, Y, So, S, et al. (2006) Simple one-week method to construct gene-targeting vectors: application to production of human knockout cell lines. Biotechniques 41: 311-316) will be described as an example.

Specifically, BP recombination is first carried out between a 5' arm having attB4 and attB1 sequences at the ends (corresponding to the "DNA fragment homologous to the 5' upstream region of a target site" in the present invention) and pDONR P4-P1R, and also between a 3' arm having attB2 and attB3 sequences at the ends (corresponding to the "DNA fragment homologous to the 3' downstream region of a target site" in the present invention) and pDONR P2R-P3, so as to produce a 5'-entry clone and a 3'-entry clone, respectively. (The 5' arm and the 3' arm are preliminarily obtained by genomic PCR.)

It is recommended that a reverse primer used for amplification of the 5' arm be preliminarily designed on the exon of a target gene. As a result, an SA site (splice acceptor site) allowing for natural splicing from an upstream exon to a selection marker gene (or an exon into which this marker gene has been inserted) on the target gene can be included within the 5' arm. The SA site is not limited to the SA sequence of the target gene, and another SA site may also be used.

It is also recommended that a restriction site for vector linearization (e.g. I-SceI, PmeI, AscI, Swal, PacI, etc.) (corresponding to the "restriction site for linearization" in the present invention) be added to the reverse primer for 3' arm amplification (or the forward primer for 5' arm amplification). As a result, restriction mapping experiments for determining restriction enzymes for linearization can be omitted.

Next, LR recombination is carried out between four components, namely, the two entry clones, pENTR IRES-Hyg prepared by introduction of a hygromycin resistance gene flanked by attL1 and attL2 sequences, and pDEST R4-R3 (Invitrogen). Only these two steps are required to complete an exon-trapping-type, substitution-type targeting vector (Figure 2).

A DNA sequence allowing for bicistronic expression (for example, an IRES (internal ribosomal entry site, which is a site for ribosomal entry in mRNA; a site derived from encephalomyocarditis virus (EMCV), etc.) sequence, a 2A peptide sequence (a 2A "self-cleaving" peptide sequence; one derived from Thosea asigna virus (TaV), etc.), IRES2, etc.) is added 5' upstream of a hygromycin resistance gene (other selection markers may also be used). Since the DNA sequence allowing for bicistronic expression is present 5' upstream of the selection marker, when gene targeting takes place, gene expression of the selection marker is achieved depending on the target gene promoter.

It is recommended that the hygromycin resistance gene be flanked with lox71 and loxP. As a result, after completion of the gene targeting, the selection marker can be removed from the genome by transient expression of Cre. However, this is not the sole means for removing the marker. Other target sequences of site-specific recombinases, such as other lox sequences or FRT sequences, may also be used.

If desired, a splice acceptor site (SA site) may be introduced into the entry clone pENTR IRES-Hyg. By introducing the splice acceptor site, a reverse primer for 5' arm amplification can be placed in an intron (not in an exon).

While the production of a targeting vector (specifically, a step of linking a 5' arm, a selection marker, and a 3' arm) has been explained above taking the case of employing the MultiSite Gateway system as an example, this is not the sole linking method to be employed. That is, it is also possible to produce targeting vectors by other molecular biological methods or by using other items (for example, general methods using restriction enzymes or DNA ligase, In-Fusion PCR Cloning, etc.). Also, as a base for targeting vector construction without the use of entry clones (namely, the Gateway system), a plasmid in which a selection marker is flanked by multiple restriction sites may be used, as these sites permit incorporation of 5' and 3' arms, as well as vector linearization.

Gene knockout cells can be produced by introducing a genetic mutation into a cell with the use of the gene targeting vector of the present invention. Such gene knockout cells can be produced by previously described methods (for example, Adachi, N, So, S, Iiizumi, S, et al. (2006) The human pre-B cell line Nalm-6 is highly proficient in gene targeting by homologous recombination. DNA Cell Biol. 25: 19-24; Adachi, N, Nishijima, H, Shibahara, K (2008) Gene targeting using the human Nalm-6 pre-B cell line. BioScience Trends. 2: 169-180; Toyoda, E, Kagaya, S, Cowell, IG, et al. (2008) NK314, a topoisomerase II inhibitor that specifically targets the alpha isoform. J. Biol. Chem. 283: 23711-23720). To explain briefly, a targeting vector is linearized with a restriction enzyme, the linearized vector is then transferred into cells according to a gene transfer method such as electroporation, and the cells are then cultured, thereby forming colonies. Subsequently, cells into which a genetic mutation has been introduced are selected, using a marker as appropriate. In order to obtain cells into which a genetic mutation has been homozygously introduced (homozygously disrupted cell line), a second gene targeting may be carried out using a different selection marker. In addition, such a selection marker can be removed by site-specific recombinase, for example, by transiently expressing Cre recombinase with the use of pBS185 plasmid. Another mutation can be introduced into the cells from which the selection marker has been removed. Examples of the cells suitable for use in gene targeting include, but are not limited to, human Nalm-6 cells, chicken DT40 cells, and mouse ES cells.

### Example 1

Hereinafter, the present invention will be described in detail by means of Examples. However, these Examples are not intended to limit the scope of the present invention.

### [Example 1]

### (Materials and Methods)

### Construction of target vector

### Materials

1. ExTaq™ polymerase (TAKARA BIO, INC.)
2. PCR primers: (for use in the amplification of the *HPRT* gene)
   Primers for amplification of the 5' arm
   (1) HPRT 5'Fw, 5'-GGGGACAACTTTGTATAGAAAAGTTGCACATCACAGGTACCATATCAGTG -3' (SEQ ID NO: 1);
   (2) HPRT 5' Rv (placed on the exon), 5'-GGGGACTGCTTTTTTGTACAAACTTGCACATCTCGAGCAAGACGTTCAGT -3' (SEQ ID NO: 2);
      Primer for amplification of the 3' arm
   (3) HPRT 3'Fw, 5'-GGGGACAGCTTTCTTGTACAAAGTGGCCTGCAGGATCACATTGTAGCCCTCTGTGTGC -3' (SEQ ID NO: 3);
   (4) HPRT 3' Rv (to which an I-SceI site serving as a restriction site for linearization has been added), 
5'-
GGGGACAACTTTGTATAATAAAGTTGCTATATTACCCTGTTATCCCTAGCGTAACTCAGGGT
AGAAATGCTACTTCAGGC -3' (SEQ ID NO: 4)
3. MultiSite Gateway (registered trademark) Three Fragment Vector Construction Kit (Invitrogen)
4. Entry clone (pENTR IRES-Hyg) into which a drug resistance gene has been incorporated
pENTR IRES-Hyg was produced by digesting the plasmid pENTR loxP (Iiizumi, S, Nomura, Y, So, S, et al. (2006) Simple one-week method to construct gene-targeting vectors: application to production of human knockout cell lines. Biotechniques 41: 311-316) with NotI, and then, successively adding lox71, IRES, Hyg, pA, and loxP to the digested plasmid. In the process, lox71 and loxP were added to the plasmid using synthetic linker DNA; IRES and pA are derived from the vector pIRES (TAKARA BIO, INC.; http://catalog.takara-bio.co.jp/product/basic_info.asp?unitid=U100004407); and Hyg is derived from pENTR lox-Hyg (Iiizumi, S, Nomura, Y, So, S, et al. (2006) Simple one-week method to construct gene-targeting vectors: application to production of human knockout cell lines. Biotechniques 41: 311-316.)
5. Destination vector (pDEST R4-R3) (Invitrogen)
6. Antibiotic-containing LB agar medium: LB agar medium containing 50 µg/ml kanamycin or 50 µg/ml ampicillin

### Protocols

1. Genomic DNA was prepared from Nalm-6 cells (Adachi, N, So, S, Iiizumi, S, et al. (2006) The human pre-B cell line Nalm-6 is highly proficient in gene targeting by homologous recombination. DNA Cell Biol. 25: 19-24) and with this DNA used as a template, PCR was carried out under the following conditions, so as to obtain an *HPRT* genomic fragment flanked with att sequences. For amplification of the 5' arm, primers (1) and (2) were used, whereas for amplification of the 3' arm, primers (3) and (4) were used. 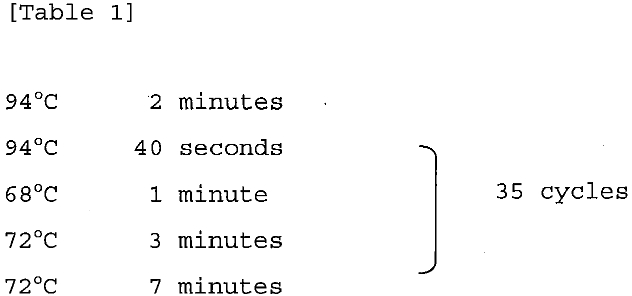
2. The obtained PCR product was purified with a commercially available kit, and was then quantified.
3. A BP recombination reaction was carried out to produce a 5'-entry clone and a 3'-entry clone (Figure 2A). The following samples were mixed in a 0.5-ml tube.

| | |
|---|---|
| pDONR P4-PIR or pDONR P2R-P3 | 50 fmoles |
| 5' or 3' arm fragment | 50 fmoles |
| Total amount | 4 µl (prepared with TE solution) |

4. 1 µl of BP Clonase II Enzyme Mix was added to the aforementioned reaction solution, and mixed well.
5. The mixture was incubated at 25°C for 4 to 5 hours.
6. 1 µl of 2 µg/µl proteinase K was added to the reaction mixture, and mixed well.
7. The resultant mixture was incubated at 37°C for 10 minutes.
8. 5 µl of the reaction solution was mixed with 50 µl of Escherichia coli competent cells to carry out transformation. After completion of a recovery culture, cells were plated on an LB agar medium containing 50 µg/ml kanamycin.
9. Ten to twenty kanamycin-resistant colonies were isolated, and plasmid DNA was then extracted from the colonies according to an alkali-SDS method. Two or three clones predicted to contain plasmids of interest were then selected by agarose gel electrophoresis. These candidate plasmids were digested with appropriate restriction enzymes, and were then subjected to agarose gel electrophoresis, whereupon they were confirmed to be the plasmids of interest.
10. The obtained 5' and 3' entry clones were purified with a commercially available kit and quantified.
11. A targeting vector (pHPRT-IRES-Hyg) was produced by an LR recombination reaction (Figure 2B). Respective samples were mixed in a 0.5-ml tube as follows.

| | |
|---|---|
| pDEST R4-R3 | 20 fmoles |
| 5'-Entry clone | 10 fmoles |
| 3'-Entry clone | 10 fmoles |
| pENTR IRES-Hyg | 10 fmoles |
| Total amount | 4 µl (prepared with TE solution) |

12. 1 µl of LR-Clonase Plus Enzyme Mix was added to the aforementioned reaction solution, and mixed well.
13. The mixture was incubated at 25°C for 16 hours.
14. 2 µl of 2 µg/µl proteinase K was added to the reaction mixture, and mixed well.
15. The resultant mixture was incubated at 37°C for 10 minutes.
16. 5 µl of the reaction solution was mixed with 50 µl of *Escherichia coli* competent cells to carry out transformation. After completion of a recovery culture, cells were plated on an LB agar medium containing 50 µg/ml ampicillin.
17. Ten to twenty ampicillin-resistant colonies were isolated, and plasmid DNA was then extracted from the colonies according to an alkali-SDS method. Two or three clones predicted to contain plasmids of interest were then selected by agarose gel electrophoresis. These candidate plasmids were digested with appropriate restriction enzymes, and were then subjected to agarose gel electrophoresis, whereupon they were confirmed to be the plasmids of interest (namely, targeting vectors).
18. The obtained targeting vectors were purified with a kit and quantified.

### Linearization of targeting vector

### Materials

1. Restriction enzyme I-SceI, 10x I-SceI reaction buffer, 10 mg/ml BSA (New England Biolabs)
2. PCI: TE saturated phenol, chloroform and isoamyl alcohol that were mixed at a ratio of 25:24:1 (stored at 4°C)
3. CI: Chloroform and isoamyl alcohol that were mixed at a ratio of 24:1 (stored at 4°C)
4. 3 M sodium acetate: 40.81 g of sodium acetate was dissolved in 80 ml of pure water. The obtained solution was adjusted to pH 5.2 with acetic acid, and the total amount of the solution was adjusted to 100 ml.
5. TE solution: 10 mM Tris-HCl buffer (pH 8.0), 0.1 mM EDTA (pH 8.0) (stored at 4°C)

### Protocols

1. The targeting vector was digested with the restriction enzyme I-SceI. Individual reagents were mixed together as follows, and the obtained mixture was then incubated at 37°C for 4 hours or more.

| | |
|---|---|
| Targeting vector | 50 µg |
| 10x I-SceI buffer | 40 µl |
| 100x BSA (10 mg/ml) | 4 µl |
| I-SceI | 15 units |
| Total amount | 400 µl (prepared with sterilized water) |

2. 40 µl of 3 M sodium acetate and 0.9 ml of ethanol were added to the reaction solution, and mixed well.
3. The mixture was centrifuged at 15,000 rpm for 5 minutes.
4. The pellet was washed with 0.5 ml of 70% ethanol three times.
5. After completion of the 3^{rd} centrifugation, supernatants were removed using a sterilized tip in a clean bench, followed by air-drying.
6. A TE solution was added to dissolve DNA (to a DNA concentration of 2 to 4 µg/µl).
7. The solution was incubated at 65°C for 15 minutes.

### Gene transfer by electroporation

### Materials

1. Growth medium: A medium prepared by adding 10% fetal bovine serum (HyClone) and 50 µM 2-mercaptoethanol to ES medium (NISSUI PHARMACEUTICAL CO., LTD.). The prepared medium was kept warm at 37°C in a hot water bath.
2. Linearized targeting vector
3. Cell Line Nucleofector Kit T (Lonza)

### Protocols

1. Human Nalm-6 cells (2 × 10⁶ cells or more) that were at a logarithmic growth phase were recovered in a 50-ml centrifugal tube.
2. The cells were centrifuged at 1,100 rpm for 5 minutes, and supernatants were gently removed.
3. 100 µl of Solution T was added to the cell mass, which was fully suspended in the solution.
4. 2 µg of the targeting vector was added to the suspension, mixed well, and transferred into a cuvette using a dropper included in the kit.
5. The cuvette was fitted onto an electroporation device (Nucleofector II, Lonza).
6. Program C-005 was executed.
7. The cells were immediately transferred into a 60-mm dish that contained 6 ml of growth medium.
8. The cells were cultured at 37°C for 20 to 24 hours.

### Colony formation

### Materials

1. 2.25 × ES medium: Individual reagents were successively dissolved in pure water as follows, and the obtained solution was fully stirred at room temperature and was sterilized by filtration (stored at 4°C).

| | |
|---|---|
| Powder ES medium | 21.8 g |
| Sodium hydrogencarbonate | 4.7 g |
| L-glutamine | 0.68 g |
| 2-mercaptoethanol | 8.1 µl |
| Total amount | 1000 ml (prepared with pure water) |

2. Fetal bovine serum (HyClone)
3. 0.33% agarose solution: 100 ml of pure water was added to 0.33 g of LE agarose (Lonza), and the obtained mixture was then sterilized in an autoclave. After completion of the sterilization, the resultant mixture was homogeneously blended before the agarose solidified, and the resulting mixture was kept warm at 40°C in a hot water bath.
4. Selective drug (100 mg/ml hygromycin B): 1 g of hygromycin B was dissolved in 10 ml of pure water, and the obtained solution was then sterilized by filtration (stored at 4°C).

### Protocols

1. 2 × ES medium (2.25 × ES medium supplemented with a quarter amount of fetal bovine serum) was prepared and then kept warm at 40°C.
2. An agarose medium was prepared (by mixing the 2 × ES medium with an equal amount of a 0.33% agarose solution and then intensively stirring the mixed solution). The prepared agarose medium was kept warm at 40°C until immediately before use.
3. 1 ml each of the transfected cells was dispensed into 90-mm dishes.
4. 40 µl of selective drug (100 mg/ml hygromycin) was added to each dish, with care taken to avoid direct contact with the cells.
5. 9 ml of the agarose medium that had been kept warm at 40°C was added to each dish, and mixed well.
6. The dishes were allowed to stand at room temperature for 20 to 30 minutes, so that the agarose was solidified.
7. The cells were cultured at 37°C for 2 to 3 weeks, so as to form colonies.

### Isolation of colonies and selection of targeted clones Materials

1. Selection medium (hygromycin B-containing medium): a growth medium supplemented with hygromycin B to a concentration of 0.4 mg/ml
2. Lysis buffer: 20 mM Tris-HCl buffer (pH 8.0), 250 mM sodium chloride, 1% SDS
3. 10 mg/ml proteinase K: 100 mg of proteinase K as dissolved in 10 ml of pure water and then sterilized by filtration (stored at -20°C)
4. Saturated NaCl solution
5. ExTaq™ polymerase
6. PCR primers: (used for confirmation of *HPRT* gene targeting; Iiizumi et al. Nucleic Acids Res., 2008, Nov; 36(19): 6333-6342.)
   HPRT-F, 5'-TGAGGGCAAAGGATGTGTTACGTG-3' (SEQ ID NO 5)
   HPRT-R, 5'-TTGATGTAATCCAGCAGGTCAGCA-3' (SEQ ID NO 6)

### Protocols

1. 0.5 ml each of selection medium was dispensed into a 48-well plate.
2. 50 to 200 colonies were picked up using a yellow or blue tip, and the colonies were then transferred into a selection medium, while pipetting was fully performed.
3. The cells were cultured at 37°C for 2 to 3 days.
4. Each culture medium was transferred into a 1.5-ml tube. After centrifugation at 3,000 to 3,500 rpm for 5 to 10 minutes, the cells were recovered.
5. After supernatants were removed, 270 µl of lysis buffer and 1 µl of 10 mg/ml proteinase K were added to the cells.
6. The mixture was incubated at 37°C overnight (or at 55°C for 1 hour).
7. 80 µl of saturated NaCl solution was added to the reaction solution, and mixed well.
8. 0.9 ml of ethanol was further added to the mixture.
9. The resultant mixture was centrifuged at 15,000 rpm at 4°C for 15 minutes.
10. The supernatant was removed and the precipitate was washed with 0.5 ml of 70% ethanol.
11. The precipitate was dissolved in 30 to 100 µl of TE solution.
12. Using the prepared genomic DNA as a template, PCR was carried out with primers HPRT-F and HPRT-R under the following conditions to screen the targeted clones.

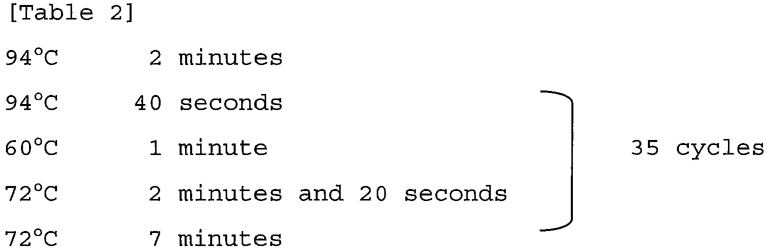

A puromycin resistance gene or a fusion gene of a β-galactosidase gene with a neomycin resistance gene can be substituted for the hygromycin resistance gene. Using such a drug resistance gene, the aforementioned operations were repeated, so as to produce a targeting vector.

In addition, an IRES2 sequence or a 2A peptide sequence can be substituted for the IRES sequence as a DNA sequence allowing for bicistronic expression. Using such a DNA sequence, the aforementioned operations were repeated, so as to produce a targeting vector.

### (Results)

The results are summarized in the following table.

**[Table 3]**

| | Targeting vector | Number of clones analyzed | Number of correctly targeted clones | Targeting efficiency (%) |
|---|---|---|---|---|
| Experiment 1 | HPRT-IRES-Puro | 17 | 13 | 76 |
| Experiment 2 | HPRT-IRES-Puro | 17 | 10 | 59 |
| Experiment 3 | HPRT-IRES-Puro | 28 | 17 | 61 |
| Experiment 4 | HPRT-IRES2-Hyg | 16 | 4 | 25 |
| Experiment 5 | HPRT-2A-Hyg | 22 | 9 | 41 |
| Experiment 6 | HPRT-2A-Puro | 5 | 5 | 100 |
| Experiment 7 | HPRT-2A-Puro | 6 | 5 | 83 |

As shown in the above results, using an exon-trapping-type targeting vector, gene targeting was successfully carried out in human Nalm-6 cells with much higher efficiency than when conventional vectors were used.

### Example 2

A gene targeting vector was produced by the same operations as in Example 1, except that the *CTIP, LIG4,* or *KU70* gene was targeted, instead of the *HPRT* gene. The gene targeting vector was subjected to linearization, transfection, colony formation and isolation, as well as selection of the targeted clones.

The results are summarized in the following table.

**[Table 4]**

| Locus | Selection marker | Targeting efficiency |
|---|---|---|
| *HPRT* | IRES-Puro | 86% (32/37) |
| | IRES-Puro | 100%(13/13) |
| | 2A-Puro | 95%(36/38) |
| | 2A-GFP-2A-Puro | 90%(19/21) |
| *CTIP* | IRES-Hygro | 69%(20/29) |
| | IRES-Hygro | 67% (6/9) |
| | IRES-Puro | 25%(1/4) |
| *LIG4* | IRES-Puro | 100%(5/5) |
| | IRES-Hygro | 80%(4/5) |
| *KU70* | IRES-Puro | 25%(1/4) |

### Example 3

Puro, Hygro, Neo or βgeo was linked downstream of an IRES, IRES2 or 2A sequence, so as to construct various drug resistance gene cassettes. A 2A-Puro gene unit was also constructed by adding 2A-EGFP upstream of 2A-Puro. With regard to IRES-Puro, IRES-Neo, IRES-Hygro and 2A-Hygro, it was desired to control the expression of the target gene with tetracycline, and thus those vectors were constructed by adding an appropriate gene or promoter necessary for this purpose. A method for constructing exon-trapping-type targeting vectors and the selection vectors thus constructed are shown in Figures 4 and 5, respectively.

All publications, patents and patent applications cited herein are incorporated herein by reference in their entirety.

### Industrial Applicability

The present invention is effective for enabling a wider and/or more efficient use of gene knockout or gene trapping in the fields of basic biology, medicine, and agriculture & livestock industries.

### Sequence Listing Free Text

< SEQ ID NO: 1 >
   SEQ ID NO: 1 shows the DNA sequence of a forward primer for amplification of a 5' arm that targets the human *HPRT* gene.
   5'- GGGGACAACTTTGTATAGAAAAGTTGCACATCACAGGTACCATATCAGTG -3'
   (The underlined portion indicates the attB4 sequence.)
< SEQ ID NO: 2 >
   SEQ ID NO: 2 shows the DNA sequence of a reverse primer for amplification of a 5' arm that targets the human *HPRT* gene. 5'- GGGGACTGCTTTTTTGTACAAACTTGCACATCTCGAGCAAGACGTTCAGT -3'
   (The underlined portion indicates the attB1 sequence.)
< SEQ ID NO: 3 >
   SEQ ID NO: 3 shows the DNA sequence of a forward primer for amplification of a 3' arm that targets the human *HPRT* gene.
   5'- GGGGACAGCTTTCTTGTACAAAGTGGCCTGCAGGATCACATTGTAGCCCTCTGTGTGC -3'
   (The underlined portion indicates the attB2 sequence.)
< SEQ ID NO: 4 >
   SEQ ID NO: 4 shows the DNA sequence of a reverse primer for amplification of a 3' arm that targets the human *HPRT* gene. 
5'- GGGGACAACTTTGTATAATAAAGTTGCTATATTACCCTGTTATCCCTAGCGTAACTCA
GGGTAGAAATGCTACTTCAGGC -3'
 (The underlined portion indicates the attB3 sequence.)
< SEQ ID NO: 5 >
   SEQ ID NO: 5 shows the DNA sequence of a PCR primer (HPRT-F) for confirmation of gene targeting.
   HPRT-F, 5'-TGAGGGCAAAGGATGTGTTACGTG-3'
< SEQ ID NO: 6 >
   SEQ ID NO: 6 shows the DNA sequence of a PCR primer (HPRT-R) for confirmation of gene targeting.
   HPRT-R, 5'-TTGATGTAATCCAGCAGGTCAGCA-3'

## Claims

1. A method for producing a gene targeting vector, comprising linking a DNA fragment homologous to a 5' upstream region of a target site, a selection marker having a DNA sequence allowing for bicistronic expression present 5' upstream thereof, and a DNA fragment homologous to a 3' downstream region of the target site.

2. The method according to claim 1, wherein the DNA fragment homologous to the 5' upstream region of the target site comprises a splice acceptor site.

3. The method according to claim 1 or 2, wherein the DNA fragment homologous to the 3' downstream region of the target site or the DNA fragment homologous to the 5' upstream region of the target site comprises a restriction site(s) for linearization.

4. A gene targeting vector, wherein a DNA sequence allowing for bicistronic expression is present 5' upstream of a selection marker.

5. The vector according to claim 4, wherein the selection marker has a poly A sequence but does not have a promoter.

6. The vector according to claim 4 or 5, wherein the selection marker is flanked with target sequences of a site-specific recombinase.

7. The vector according to any one of claims 4 to 6, further comprising a splice acceptor site.

8. The vector according to any one of claims 4 to 7, further comprising a restriction site(s) for linearization.

9. A method for producing a gene knockout cell, comprising introducing a genetic mutation into a cell with the use of the gene targeting vector according to any one of claims 4 to 8.

10. A vector comprising a selection marker to be used for the production of a gene targeting vector, wherein a DNA sequence allowing bicistronic expression is incorporated 5' upstream of the selection marker.

11. The vector according to claim 10, further comprising a splice acceptor site.

12. A vector comprising a selection marker to be used for the production of a gene targeting vector, further comprising sites for incorporation of a DNA fragment homologous to a 5' upstream region of a target site and a DNA fragment homologous to a 3' downstream region of the target site, and a restriction site(s) for linearization.
